# EUROPEAN PATENT APPLICATION

(11) **EP 2 762 188 A1**
(43) Date of publication of application: **06.08.2014**
(21) Application number: 14152816.6
(22) Date of filing: 28.01.2014
(51) Int. Cl.: A61M 25/00, A61M 25/09

(54) **Slitted pipe and guidewire including the same**

(30) Priority: 31.01.2013 JP 2013016965
(71) Applicant: ASAHI INTECC CO., LTD., Nagoya-shi, Aichi 463-0024 (JP)
(72) Inventor: Kimura, Kazuyuki, Nagoya-shi, Aichi 463-0024 (JP)
(74) Representative: Winter, Brandl, Fürniss, Hübner, Röss, Kaiser, Polte - Partnerschaft

(57) **Abstract**

A slitted pipe includes a slit pattern (15) provided in an area except for both end portions in a longitudinal direction of the slitted pipe to extend in the longitudinal direction in a helical shape, and a plurality of girders (19a to 19f) provided on the slit pattern(15) to divide the slit pattern (15) into a plurality of slits (15').

## Description

### BACKGROUND OF THE INVENTION

### 1. Field of the Invention

The present invention relates to a slitted pipe having a slit in a surface, and a guidewire including the slitted pipe.

### 2. Description of the Related Art

Various guidewires have hitherto been proposed to guide a medical instrument, for example, a catheter to be inserted in a tubular organ such as a blood vessel, a digestive tract, or a ureter, or a body tissue.

For example, U.S. Patent No. 5,345,945 (Patent Document 1) describes that an inner coil is fixed to a distal end of a guidewire by a brazing part (see Fig. 1B of Patent Document 1). Patent Document 1 also describes that, when a distal end of an inner coil is located apart from a distal end of a guidewire toward a proximal end, the inner coil is fixed to a brazing part (see Figs. 5A and 5B of Patent Document 1).

In contrast, Japanese Unexamined Patent Application Publication (Translation of PCT Application) No. 2007-514458 (Patent Document 2) describes a reinforcing member (tubular member) having a plurality of cuts formed by grooves or one helical cut (see Figs. 3A and 3B of Patent Document 2).

In the guidewire described in Patent Document 1, end portions of a wire that forms the inner coil may get loose. Hence, when the guidewire is produced, it is necessary to braze the end portions of the inner coil to a core wire so that the end portions will not get loose. This causes troublesomeness in production.

Since the inner coil is helically wound around the core wire, when the end portions of the inner coil is brazed, the inner coil needs to be brazed over the entire circumferential cross section. The inner coil is brazed to the core wire also with attention to this point.

In the reinforcing member illustrated in Fig. 3B of Patent Document 2, since a plurality of grooves are concentrically formed in the tubular member, rigidity (especially, flexibility) of the reinforcing member can be adjusted. However, it is difficult to expect enhancement of torque transmissibility to a distal end of a guidewire when the manipulator rotates a proximal end of the guidewire and an increase in thrust force of the guidewire in a lesion.

Further, in the reinforcing member illustrated in Fig. 3A of Patent Document 2, only one helical cut is provided in the tubular member. In a sense, the cut is a factor for formation of play when the guidewire is rotated. Hence, it is necessary to set the width of the cut in consideration of play of the guidewire and to minimize expansion and contraction of the tubular member in an axial direction.

For example, when the guidewire enters a chronic total occlusion lesion (hereinafter referred to as a CTO lesion), the distal end of the guidewire does sometimes not advance into the CTO lesion even when the manipulator repeats operations of pushing and pulling the proximal end of the guidewire. Since it is extremely important to enhance torque transmissibility of the guidewire in the CTO lesion, measures have recently been tried to further advance the distal end of the guidewire into the CTO lesion by enhancing torque transmissibility in rotation of the guidewire in at least one direction.

### SUMMARY OF THE INVENTION

The present invention has been made in view of the above circumstances, and an object of the invention is to provide a slitted pipe that enhances torque transmissibility in rotation in at least one direction and prevents expansion and contraction in an axial direction, a slitted pipe that allows rigidity to be adjusted easily, a slitted pipe that enhances torque transmissibility in a well-balanced manner in a circumferential cross section, and a guidewire that uses the slitted pipe and that can be produced easily.

A slitted pipe according to a first aspect includes a slit pattern provided in an area except for both end portions in a longitudinal direction of the slitted pipe to extend in the longitudinal direction in a helical shape, and a plurality of girders provided on the slit pattern to cross over the slit pattern. I.e., the girders divide the slit pattern into plurality of slits.

Thus, the slit pattern comprises a plurality of slits divided by the girders and arranged on the same helical shape in a row.

According to the slitted pipe of the first aspect, the slitted pipe itself can be made flexible, and the end portions of the slitted pipe can be prevented from getting loose. Moreover, torque transmissibility in rotation in at least one direction can be enhanced, and expansion and contraction of the slitted pipe in the axial direction can be prevented.

A guidewire according to a second aspect of the present invention includes a core shaft, and the above slitted pipe covering a distal end portion of the core shaft.

According to the guidewire of the second aspect, the guidewire can be easily produced, and torque transmissibility of the guidewire in rotation in at least one rotation can be enhanced.

A guidewire according to a third aspect of the present invention includes a core shaft, and the above slitted pipe covering a distal end portion of the core shaft such that the other end of the slitted pipe is located on a distal side of the core shaft.

According to the guidewire of the third aspect, the guidewire can be easily produced, and torque transmissibility of the guidewire in rotation in at least one direction can be enhanced. Moreover, flexibility of the distal end portion of the guidewire can be further enhanced by the slitted pipe.

A guidewire according to a fourth aspect of the present invention includes a core shaft, a coil body covering a distal end portion of the core shaft, and the above slitted pipe disposed in the coil body to cover the distal end portion of the core shaft.

According to the guidewire of the fourth aspect, the guidewire can be easily produced, and torque transmissibility of the guidewire in rotation in at least one direction can be enhanced. Moreover, flexibility of the guidewire can be adjusted by the slitted pipe disposed therein while the outer diameter of the coil body is maintained.

A guidewire according to a fifth aspect of the present invention includes a core shaft, a coil body covering a distal end portion of the core shaft, and the above slitted pipe disposed in the coil body to cover the distal end portion of the core shaft such that the other end of the slitted pipe is located on a distal side of the core shaft.

According to the guidewire of the fifth aspect, the guidewire can be easily produced, and torque transmissibility of the guidewire in rotation in at least one direction can be enhanced. Moreover, flexibility of the distal end portion of the guidewire can be adjusted by the slitted pipe disposed therein while the outer diameter of the coil body is maintained, and flexibility of the distal end portion of the guidewire can be enhanced.

### BRIEF DESCRIPTION OF THE DRAWINGS

Fig. 1A is an overall perspective view of a slitted pipe according to a first embodiment of the present invention, and Fig. 1B is a cross-sectional view taken along line IB-IB of Fig. 1A;
Fig. 2A is an overall perspective view of a slitted pipe according to a second embodiment of the present invention, and Fig. 2B is a cross-sectional view taken along line IIB-IIB of Fig. 2A;
Fig. 3A is an overall perspective view of a slitted pipe according to a third embodiment of the present invention, Fig. 3B is a cross-sectional view taken along line IIIB-IIIB of Fig. 3A, and Fig. 3C illustrates the length of a slit along a helical shape and a length of girders along a helical shape per unit length;
Fig. 4A is an overall perspective view of a slitted pipe according to a fourth embodiment of the present invention, and Fig. 4B is a cross-sectional view taken along line IVB-IVB of Fig. 4A;
Fig. 5A is an overall perspective view of a slitted pipe of according to a fifth embodiment of the present invention, and Fig. 5B is a cross-sectional view taken along line VB-VB of Fig. 5A;
Fig. 6A is an overall perspective view of a slitted pipe according to a sixth embodiment of the present invention, Fig. 6B is a cross-sectional view taken along line VIB-VIB of Fig. 6A, Fig. 6C is a cross-sectional view taken along line VIC-VIC of Fig. 6A, and Fig. 6D is a cross-sectional view taken along line VID-VID of Fig. 6A;
Fig. 7A is an overall perspective view of a slitted pipe according to a seventh embodiment of the present invention, Fig. 7B is a cross-sectional view taken along line VIIB-VIIB of Fig. 7A, Fig. 7C is a cross-sectional view taken along line VIIC-VIIC of Fig. 7A, and Fig. 7D is a cross-sectional view taken along line VIID-VIID of Fig. 7A;
Fig. 8A is an overall perspective view of a slitted pipe according to an eighth embodiment of the present invention, Fig. 8B is a cross-sectional view taken along VIIIB-VIIIB of Fig. 8A, Fig. 8C is a cross-sectional view taken along line VIIIC-VIIIC of Fig. 8A, and Fig. 8D is a cross-sectional view taken along line VIIID-VIIID of Fig. 8A;
Fig. 9A is an overall perspective view of a slitted pipe according to a ninth embodiment of the present invention, Fig. 9B is a cross-sectional view taken along line IXB-IXB of Fig. 9A, Fig. 9C is a cross-sectional view taken along line IXC-IXC of Fig. 9A, and Fig. 9D is a cross-sectional view taken along line IXD-IXD of Fig. 9A;
Fig. 10A is an overall perspective view of a slitted pipe according to a tenth embodiment of the present invention, and Fig. 10B is a cross-sectional view taken along line XB-XB of Fig. 10A;
Fig. 11A is an overall perspective view of a slitted pipe according to an eleventh embodiment of the present invention, and Fig. 11B is a cross-sectional view taken along line XIB-XIB of Fig. 11A;
Fig. 12 is an overall view of a guidewire according to a twelfth embodiment of the present invention;
Fig. 13 is an overall view of a guidewire according to a thirteenth embodiment of the present invention; and
Fig. 14 is an overall view of a guidewire according to a fourteenth embodiment of the present invention.

### DESCRIPTION OF THE PREFERRED EMBODIMENTS

First, a slitted pipe according to the present invention will be described on the basis of preferred embodiments illustrated in the drawings.

### First Embodiment

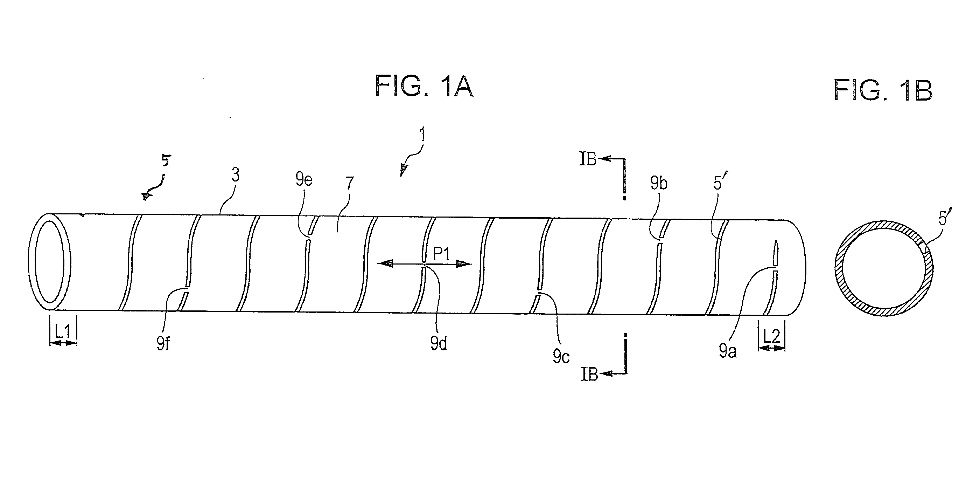

Fig. 1A is an overall perspective view of a slitted pipe according to a first embodiment of the present invention, and Fig. 1B is a cross-sectional view taken along line IB-IB of Fig. 1A.

In Figs. 1A and Figs. 2A to 11B described below, for easy understanding, the slitted pipe is schematically illustrated as a whole while being shortened in a longitudinal direction. Hence, overall dimensions of the slitted pipe are different from actual ones.

Referring to Figs. 1A and 1B, in a slitted pipe 1 of the first embodiment, one slit pattern 5 comprising a plurality of slit 5' and extending in a helical shape in a longitudinal direction of a tubular member 3 is formed in a surface of the tubular member 3 by laser machining or etching such that the slit 5' penetrates the tubular member 3.

The slitted pipe 1 also includes a belt-like helical portion 7 defined by the slit pattern 5, and girders provided on the helical slit pattern 5 to divide the slit pattern 5 into a plurality of slits 5', that is, girders 9 (9a, 9b, 9c, 9d, 9e, 9f, ...) provided on the helical slit pattern 5 extending in the longitudinal direction of the tubular member 3 divide the slit pattern 5 into a plurality of slits 5'. According to the slitted pipe 1, since a plurality of girders 9 are provided on the helical slit pattern 5 to cross over the slit pattern 5, expansion and contraction of the slitted pipe 1 in an axial direction are prevented, and torque transmissibility of the slitted pipe 1 can be enhanced.

Preferably, the girders adjacent in the longitudinal direction are arranged while being shifted from each other by a predetermined angle in cross section per predetermined distance in the longitudinal direction. This can enhance torque transmissibility of the slitted pipe itself in a well-balanced manner in the circumferential cross section.

While the girders 9 are shaped like a rectangle in the first embodiment, the rectangle may extend in a direction orthogonal to the helical shape, or may extend in a direction P1 parallel to a longitudinal axis of the tubular member 3. When the girders 9 are shaped like a rectangle extending in the direction orthogonal to the helical shape, the rectangular shape can be easily formed, for example, by turning on and off the output from a laser. When the girders 9 are shaped like a rectangle extending in the direction P1 parallel to the longitudinal axis of the tubular member 3, expansion and contraction of the slitted pipe 1 in the axial direction can be prevented further.

In the first embodiment, the slit pattern 5 is not provided within a distance L1 from a left end of the slitted pipe 1 and a distance L2 from a right end of the slitted pipe 1. Therefore, the slitted pipe 1 is not divided by the slit pattern 5, but can be handled as one piece. This allows the slitted pipe 1 to be handled easily.

Since the slitted pipe 1 has the slit pattern 5 in its surface, flexibility thereof can be enhanced. Hence, when the slitted pipe 1 is inserted in a blood vessel of a patient, perforation of the blood vessel can be prevented.

Since the slitted pipe 1 has the helical slit pattern 5 in its surface, play is formed when rotational force applied to one end of the slitted pipe 1 is transmitted to the other end. Hence, even if great force is suddenly applied to one end of the slitted pipe 1, the force can be prevented from being immediately transmitted to the other end. Thus, an accident can be avoided.

Since the slitted pipe 1 has the helical slit pattern 5 in its surface, even if one end of the slitted pipe 1 is locked for any reason, torque transmissibility of the slitted pipe 1 to the other end can be enhanced by further rotating the slitted pipe 1. This increases the possibility that the slitted pipe 1 will be unlocked.

In the first embodiment, the slit pattern 5 extends in a left-handed helical shape in the longitudinal direction of the tubular member 3. Hence, torque transmissibility to the other end of the slitted pipe 1 can be enhanced by rotating one end of the slitted pipe 1 in a direction to narrow the slit 5', that is, in a rightward direction.

While the material of the slitted pipe 1 is not particularly limited, stainless steel (SUS304) is used in the first embodiment. Alternatively, a superelastic alloy, such as a Ni-Ti alloy, can be used.

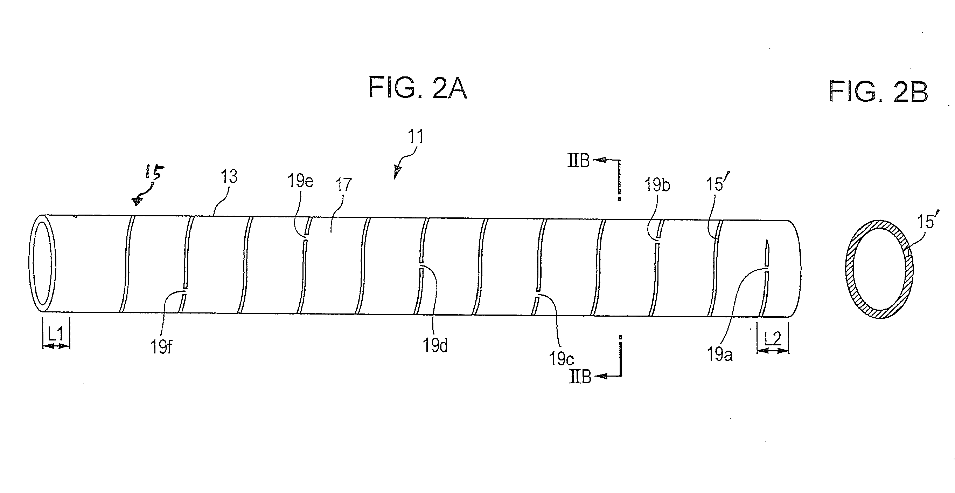

While the slitted pipe 1 of the first embodiment is shaped like a hollow cylinder, it may have other shapes. For example, the slitted pipe 1 may be shaped like a prism that is polygonal, such as triangular, quadrangular, hexagonal, or octagonal, in cross section. Alternatively, the slitted pipe 1 may be elliptical in cross section, as illustrated in Fig. 2B.

However, as described above, when the slitted pipe 1 is shaped like a hollow cylinder as in the first embodiment, torque transmissibility to the other end of the slitted pipe 1 when rotational force is applied to one end can be further enhanced because of the relationship with the helical slit pattern provided in the surface of the tubular member.

### Second Embodiment

Fig. 2A is an overall perspective view of a slitted pipe according to a second embodiment of the present invention, and Fig. 2B is a cross-sectional view taken along line IIB-IIB of Fig. 2A.

Referring to Figs. 2A and 2B, a slitted pipe 11 according to the second embodiment is similar to the slitted pipe 1 of the above-described first embodiment except that the cross section of the slitted pipe 11 is elliptical. In the slitted pipe 11, one helical slit pattern 15 extending in a longitudinal direction of a tubular member 13 is formed in a surface of the tubular member 13 by laser machining or etching such as to penetrate the tubular member 13.

The slitted pipe 11 also includes a belt-like helical portion 17 defined by the slit pattern 15, and girders 19a, 19b, 19c, 19d, 19e, 19f, ... provided on the helical slit pattern 15 to divide the slit pattern 15 into plurality of slits 15'.

The slitted pipe 11 can be used when it is to be curved in one direction, but is not to be curved in the other directions. For example, in a case in which the slitted pipe 11 is used in a two-dimensional space, a peculiar effect is achieved when the slitted pipe 11 is to be curved only in a direction of the short side of the elliptical cross section, but is not to be curved in a direction of the long side of the elliptical cross section.

In both longitudinal end portions of the slitted pipe 11, a slit is not formed, similarly to the above-described slitted pipe 1. That is, the slit pattern 15 is not formed within a distance L1 from a left end of the slitted pipe 11 and a distance L2 from a right end of the slitted pipe 11 in Fig. 2A. Therefore, similarly to the slitted pipe 1, the slitted pipe 11 is not divided by the slit pattern 15, but can be handled as one piece. This allows the slitted pipe 11 to be handled easily.

The slitted pipe 11 is similar to the slitted pipe 1 in the following respects. Since the slitted pipe 11 has one helical slit pattern 15 in its surface, play is formed when rotational force applied to one end of the slitted pipe 11 is transmitted to the other end. Hence, even if great force is suddenly applied to one end of the slitted pipe 11, it is prevented from being immediately transmitted to the other end. Further, since the slitted pipe 11 has one helical slit pattern 15 in its surface, even if one end of the slitted pipe 11 is locked for any reason, torque transmissibility to the other end of the slitted pipe 11 can be enhanced by further rotating the slitted pipe 11.

### Third Embodiment

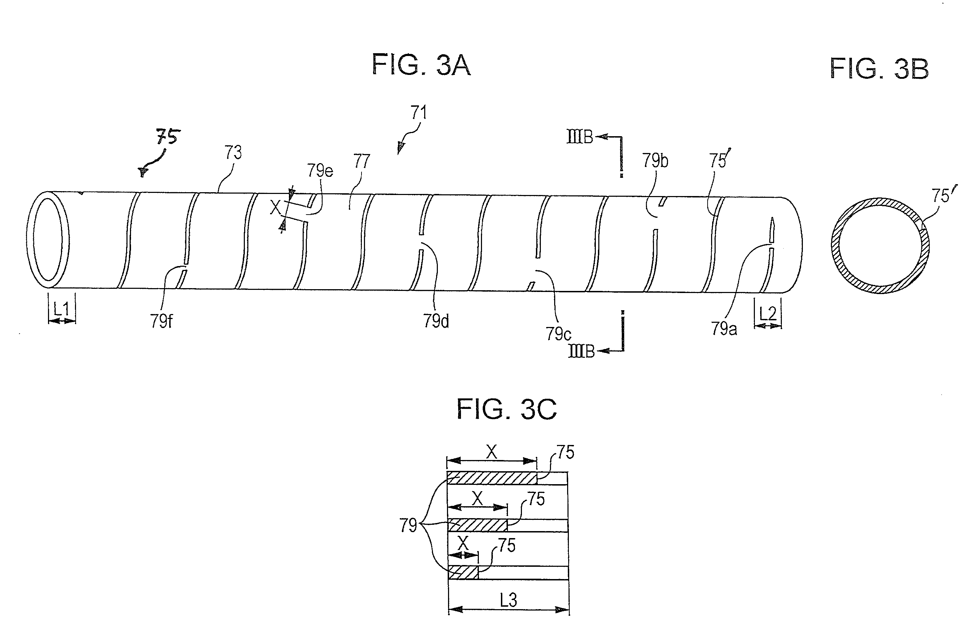

Fig. 3A is an overall perspective view of a slitted pipe according to a third embodiment of the present invention, Fig. 3B is a cross-sectional view taken along line IIIB-IIIB of Fig. 3A, and Fig. 3C illustrates the length of a slit along a helical shape and the length of girders along the helical shape per unit length.

Referring to Figs. 3A to 3C, a slitted pipe 71 according to the third embodiment is different from the first and second embodiments in that the length of girders along the helical shape differs according to rigidity of the slitted pipe 71 in a longitudinal direction. That is, the slitted pipe 71 includes one helical slit pattern 75, a belt-like helical portion 77 defined by the slit pattern 75, and girders 79 (79a, 79b, 79c, 79d, 79e, 79f, ...) provided on the helical slit pattern 75 to divide the slit pattern 75 into a plurality of slits 75'. A length X of the girders 79a, 79b, 79c, 79d, and 79e differs according to rigidity of the slitted pipe 71.

More specifically, the length X of the girders is long when rigidity of the slitted pipe 71 is high (hard), and is short when rigidity of the slitted pipe 71 is low (soft).

In the third embodiment, four regions, that is, a low-rigidity region, a high-rigidity region, a middle-rigidity region, and a low-rigidity region are set in this order from a right side of the slitted pipe 71 in Fig. 3A.

Preferably, as for the length of the girders along the helical shape, as illustrated in Fig. 3C, the length of the girders and the length of the slits 75' are considered in combination. In Fig. 3C, the helical shape of the slit 75' provided in the surface of the slitted pipe 71 is converted into a linear shape. That is, the sums of the lengths of all girders 79 and the length of the slits 75' are fixed at L3. Rigidity of the slitted pipe 71 in the longitudinal direction is changed by changing the ratio of the length (X) along the helical shape of the girders 79 to the length (L3-X) along the helical shape of the slit 75' per unit length L3. According to this, it is possible to easily set a program for producing the slitted pipe 71 and to easily produce the slitted pipe 71. It is also possible to easily set or change rigidity of the slitted pipe 71 in the longitudinal direction.

Similarly to the slitted pipe 1 and the slitted pipe 11 described above, the slit pattern 75 is not formed in both longitudinal end portions of the slitted pipe 71, that is, the slit pattern 75 is not formed within a distance L1 from a left end of the slitted pipe 71 and a distance L2 from a right end of the slitted pipe 71 in Fig. 3A. Therefore, the slitted pipe 71 is not divided by the slit pattern 75, but can be handled as one piece. This allows the slitted pipe 71 to be handled easily.

Since the slitted pipe 71 has the helical slit pattern 75 in its surface, play is formed when rotational force applied to one end of the slitted pipe 71 is transmitted to the other end. Hence, even if great force is suddenly applied to one end of the slitted pipe 71, it can be prevented from being immediately transmitted to the other end. Thus, an accident can be avoided further.

Since the slitted pipe 71 has the helical slit pattern 75 in its surface, even if one end of the slitted pipe 71 is locked for any reason, torque transmissibility to the other end of the slitted pipe 71 can be enhanced by further rotating the slitted pipe 71, similarly to the slitted pipe 1 and the slitted pipe 11.

### Fourth Embodiment

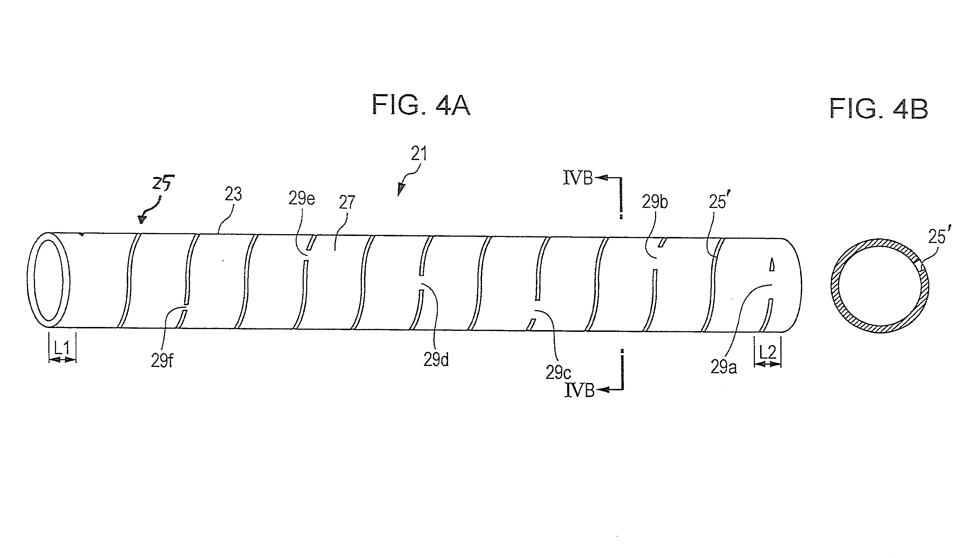

Fig. 4A is an overall perspective view of a slitted pipe according to a fourth embodiment of the present invention, and Fig. 4B is a cross-sectional view taken along line IVB-IVB of Fig. 4A.

Referring to Figs. 4A and 4B, a slitted pipe 21 of the fourth embodiment is different from the first to third embodiments in that the ratio of the length along a helical shape of girders to the length along a helical shape of a slit decreases from one end toward the other end of the slitted pipe 21 in the longitudinal direction. That is, the slitted pipe 21 includes a helical slit pattern 25 extending in a longitudinal direction of a tubular member 23, a belt-like helical portion 27 defined by the slit pattern 25, and girders 29 (29a, 29b, 29c, 29d, 29e, 29f, ...) provided on the helical slit pattern 25 to divide the slit pattern 25 into a plurality of slits 25'. The ratio of the length along the helical shape of the girders 29a, 29b, 29c, 29d, and 29e to the length along the helical shape of the slit 25' decreases from one end (a right end in Fig. 4A) toward the other end (a left end in Fig. 4A) of the slitted pipe 21 in the longitudinal direction.

According to this slitted pipe 21, flexibility at the other end (left end in Fig. 4A) of the slitted pipe 21 can be enhanced.

In the fourth embodiment, the length of the girders 29 and the length of the slit 25' are also preferably considered in combination. In this case, preferably, the sums of the lengths of the girder 29 and the length of the slit 25' is set to be equal, and rigidity of the slitted pipe 21 in the longitudinal direction is changed by changing the ratio of the length along the helical shape of the girders 29 to the length along the helical shape of the slit 25' per unit length. According to this, it is possible to easily set a program for producing the slitted pipe 21 and to easily produce the slitted pipe 21. It is also possible to easily set or change rigidity of the slitted pipe 21 in the longitudinal direction.

Similarly to the slitted pipe 1, the slitted pipe 11, and the slitted pipe 71 described above, the slit pattern 25 is not formed in both longitudinal end portions of the slitted pipe 21, that is, the slit pattern 25 is not formed within a distance L1 from a left end of the slitted pipe 21 and a distance L2 from a right end of the slitted pipe 21 in Fig. 4A. Therefore, the slitted pipe 21 is not divided by the slit pattern 25, but can be handled as one piece. This allows the slitted pipe 21 to be handled easily.

Since the slitted pipe 21 has the helical slit pattern 25 in its surface, play is formed when rotational force applied to one end of the slitted pipe 21 is transmitted to the other end. Hence, even if great force is suddenly applied to one end of the slitted pipe 21, it can be further prevented from being immediately transmitted to the other end. Thus, an accident can be avoided further.

Since the slitted pipe 21 has the helical slit pattern 25 in its surface, even if one end of the slitted pipe 21 is locked for any reason, torque transmissibility to the other end of the slitted pipe 21 can be enhanced by further rotating the slitted pipe 21, similarly to the slitted pipe 1, the slitted pipe 11, and the slitted pipe 71.

### Fifth Embodiment

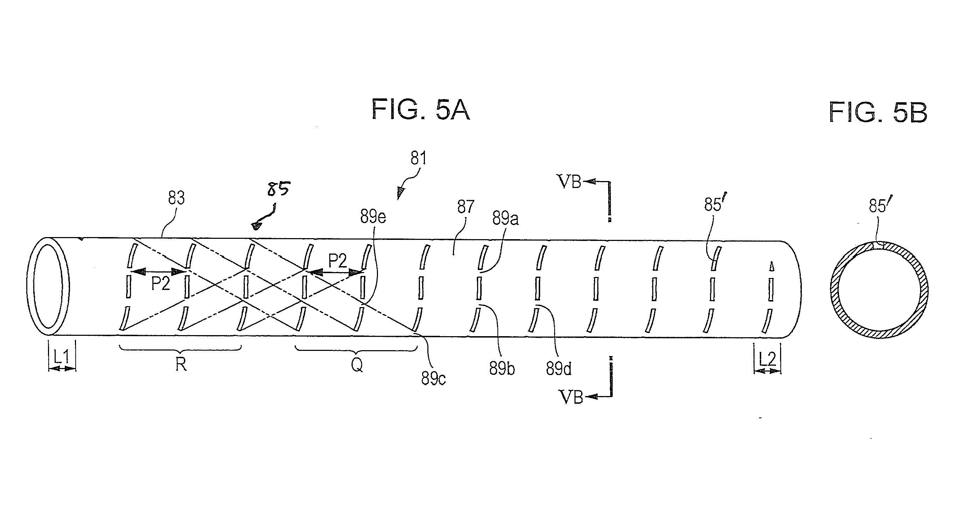

Fig. 5A is an overall perspective view of a slitted pipe according to a fifth embodiment of the present invention, and Fig. 5B is a cross-sectional view taken along line VB-VB of Fig. 5A.

Referring to Figs. 5A and 5B, a slitted pipe 81 of the fifth embodiment includes one helical slit pattern 85 extending in a longitudinal direction of a tubular member 83, a belt-like helical portion 87 defined by the slit pattern 85, and girders 89 (89a, 89b, 89c, 89d, 89e, ...) provided on the helical slit pattern 85 to divide the slit pattern 85 into a plurality of slits 85'. Unlike the above-described first embodiment, these girders 89 are arranged on a surface of the slitted pipe 81 to form a helical shapes Q or a helical shapes R different from a helical shape of the slit pattern 85. According to this slitted pipe 81, torque transmissibility can be enhanced further.

In the fifth embodiment, the slit pattern 85 is also not formed within a distance L1 from a left end of the slitted pipe 81 and a distance L2 from a right end of the slitted pipe 81. Hence, the slitted pipe 81 is not divided by the slit pattern 85, but can be handled as one piece. This allows the slitted pipe 81 to be handled easily.

The slitted pipe 81 is similar to the slitted pipe 1, the slitted pipe 11, the slitted pipe 21, and the slitted pipe 71 in that, even if great force is suddenly applied to one end of the slitted pipe 81, it can be further prevented from being immediately transmitted to the other end and in that torque transmissibility to the other end of the slitted pipe 81 can be enhanced by further rotating the slitted pipe 81.

While the girders 89 in the fifth embodiment are shaped like a rectangle, the rectangle may extend in a direction orthogonal to the helical shape, or may extend in a direction P2 parallel to a longitudinal axis of the tubular member 83. When the girders 89 are shaped like a rectangle extending in the direction orthogonal to the helical shape, the rectangular shape can be easily formed, for example, by turning on and off the output from a laser. When the girders 89 are shaped like a rectangle extending in the direction P2 parallel to the longitudinal axis of the tubular member 83, expansion and contraction of the slitted pipe 81 in the axial direction can be prevented further.

### Sixth Embodiment

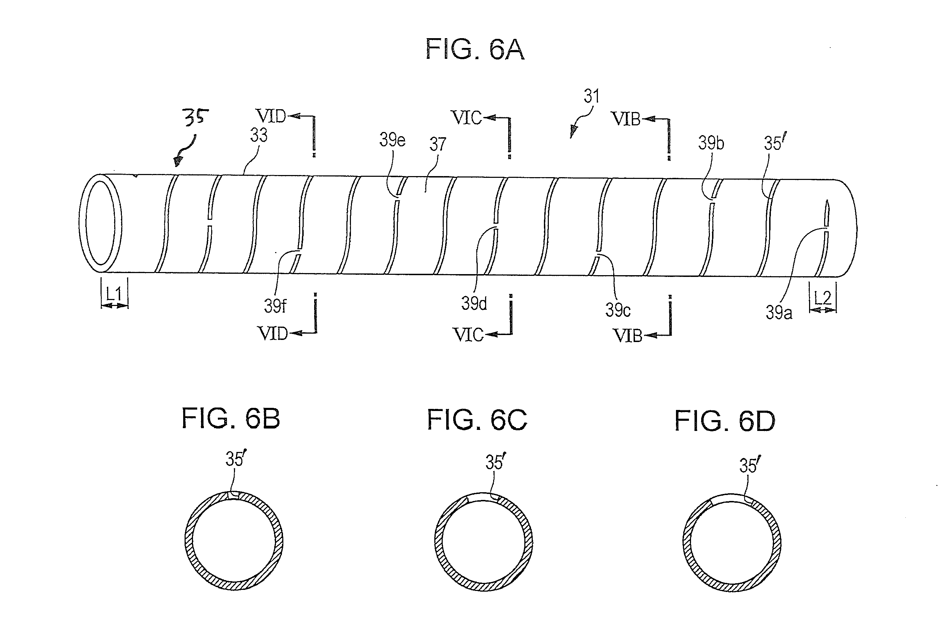

Fig. 6A is an overall perspective view of a slitted pipe according to a sixth embodiment of the present invention, Fig. 6B is a cross-sectional view taken along line VIB-VIB of Fig. 6A, Fig. 6C is a cross-sectional view taken along line VIC-VIC of Fig. 6A, and Fig. 6D is a cross-sectional view taken along line VID-VID of Fig. 6A.

Referring to Figs. 6A to 6D, a slitted pipe 31 of the sixth embodiment is different from the above-described first to fifth embodiments in that the pitch of one helical slit pattern extending in a longitudinal direction of a tubular member 33 gradually decreases from one end toward the other end of the slitted pipe 31. The slitted pipe 31 includes a helical slit pattern 35 extending in the longitudinal direction of the tubular member 33, a belt-like helical portion 37 defined by the slit pattern 35, and girders 39 (39a, 39b, 39c, 39d, 39e, 39f, ...) provided on the helical slit pattern 35 to divide the slit pattern 35 into a plurality of slits 35'. The pitch of the slit pattern 35 decreases from one end (a right end in Fig. 6A) toward the other end (a left end in Fig. 6A) of the slitted pipe 31 in the longitudinal direction. According to this slitted pipe 31, flexibility at the other end (left end in Fig. 6A) of the slitted pipe 31 can be enhanced. Therefore, when the slitted pipe 31 is inserted in a blood vessel of a patient, perforation of the blood vessel can be prevented further.

In the sixth embodiment, the helical slit pattern 35 is also not formed in both longitudinal end portions of the slitted pipe 31. That is, the slit pattern 35 is not formed within a distance L1 from a left end of the slitted pipe 31 and a distance L2 from a right end of the slitted pipe 31 in Fig. 6A. Therefore, the slitted pipe 31 is not divided by the slit pattern 35, but can be handled as one piece. This allows the slitted pipe 31 to be handled easily.

The slitted pipe 31 is similar to the slitted pipe 1, the slitted pipe 11, the slitted pipe 21, the slitted pipe 71, and the slitted pipe 81 in that, even if great force is suddenly applied to one end of the slitted pipe 31, it can be further prevented from being immediately transmitted to the other end and in that torque transmissibility to the other end of the slitted pipe 31 can be enhanced by further rotating the slitted pipe 31.

### Seventh Embodiment

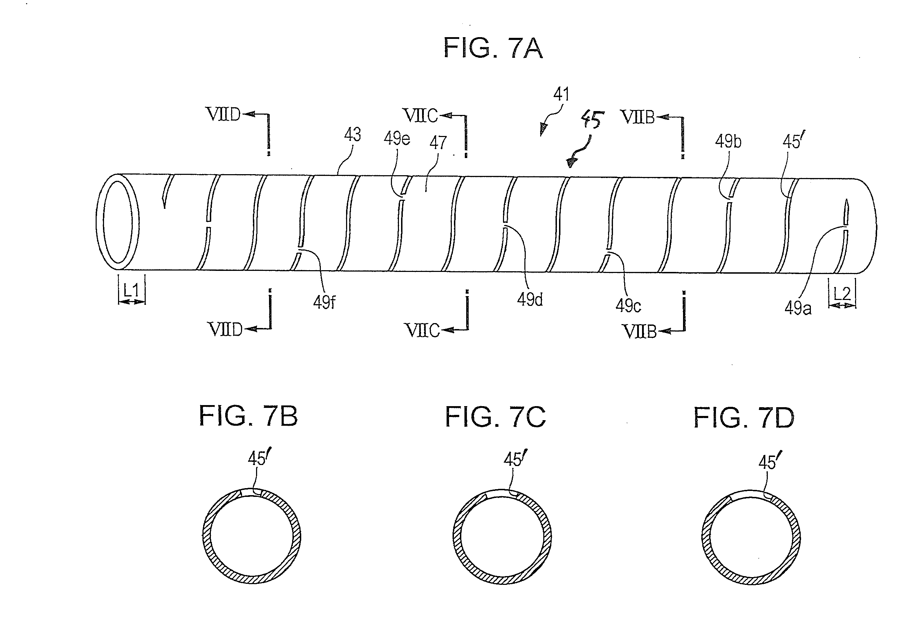

Fig. 7A is an overall perspective view of a slitted pipe according to a seventh embodiment of the present invention, Fig. 7B is a cross-sectional view taken along line VIIB-VIIB of Fig. 7A, Fig. 7C is a cross-sectional view taken along line VIIC-VIIC of Fig. 7A, and Fig. 7D is a cross-sectional view taken along line VIID-VIID of Fig. 7A.

Referring to Figs. 7A to 7D, a slitted pipe 41 of the seventh embodiment is different from the above-described first to sixth embodiments in that the pitch of one helical slit pattern extending in a longitudinal direction of a tubular member 43 decreases in a stepwise manner from one end toward the other end. That is, the slitted pipe 41 includes a helical slit pattern 45 extending in the longitudinal direction of the tubular member 43, a belt-like helical portion 47 defined by the slit pattern 45, and girders 49a, 49b, 49c, 49d, 49e, 49f, ... provided on the helical slit pattern 45 to divide the slit pattern 45 into a plurality of slits 45'. The pitch of the slit pattern 45 decreases in three steps from one end (a right end in Fig. 7A) toward the other end (a left end in Fig. 7A) of the slitted pipe 41 in the longitudinal direction. According to this slitted pipe 41, flexibility at the other end (left end) of the slitted pipe 41 can be enhanced. Hence, when the slitted pipe 41 is inserted in a blood vessel of a patient, perforation of the blood vessel can be further prevented. Moreover, the base point can be easily formed when the slitted pipe 41 is curved.

In the seventh embodiment, the slit pattern 45 is also not formed within a distance L1 from the left end of the slitted pipe 41 and a distance L2 from the right end of the slitted pipe 41. Hence, the slitted pipe 41 is not divided by the slit pattern 45, but can be handled as one piece. This allows the slitted pipe 41 to be handled easily.

The slitted pipe 41 is similar to the slitted pipe 11, the slitted pipe 21, the slitted pipe 31, the slitted pipe 71, and the slitted pipe 81 in that, even if great force is suddenly transmitted to one end of the slitted pipe 41, it can be further prevented from being immediately transmitted to the other end and in that torque transmissibility to the other end of the slitted pipe 41 can be enhanced by further rotating the slitted pipe 41.

### Eighth Embodiment

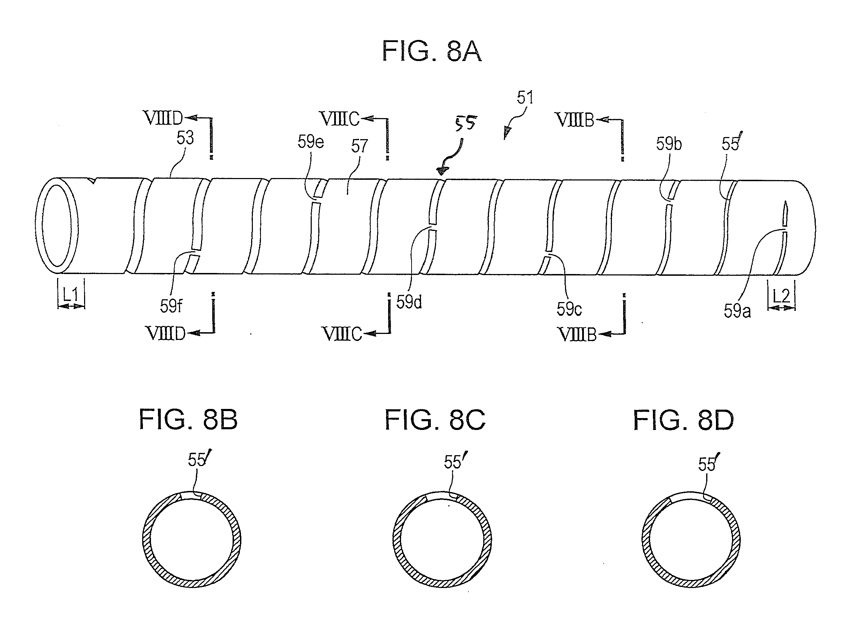

Fig. 8A is an overall perspective view of a slitted pipe according to an eighth embodiment of the present invention, Fig. 8B is a cross-sectional view taken along line VIIIB-VIIIB of Fig. 8A, Fig. 8C is a cross-sectional view taken along line VIIIC-VIIIC of Fig. 8A, and Fig. 8D is a cross-sectional view taken along line VIIID-VIIID of Fig. 8A.

Referring to Figs. 8A to 8D, a slitted pipe 51 of the eighth embodiment is different from the above-described first to seventh embodiments in that the slit width of one helical slit pattern extending in a longitudinal direction of a tubular member 53 gradually increases from one end toward the other end. That is, the slitted pipe 51 includes a helical slit pattern 55 extending in the longitudinal direction of the tubular member 53, a belt-like helical portion 57 defined by the slit pattern 55, and girders 59a, 59b, 59c, 59d, 59e, 59f, ... provided on the helical slit pattern 55 to divide the slit pattern 55 into a plurality of slits 55'. The width of the slit 55' gradually increases from one end (a right end in Fig. 8A) toward the other end (a left end in Fig. 8A) of the slitted pipe 51. According to this slitted pipe 51, flexibility at the other end (left end) of the slitted pipe 51 can be enhanced. Hence, when the slitted pipe 51 is inserted in a blood vessel of a patient, perforation of the blood vessel can be prevented further.

In the eighth embodiment, the slit pattern 55 is also not formed within a distance L1 from the left end of the slitted pipe 51 and a distance L2 from the right end of the slitted pipe 51. Hence, the slitted pipe 51 is not divided by the slit pattern 55, but can be handled as one piece. This allows the slitted pipe 51 to be handled easily.

The slitted pipe 51 is similar to the slitted pipe 1, the slitted pipe 11, the slitted pipe 21, the slitted pipe 31, the slitted pipe 41, the slitted pipe 71, and the 81 in that, even if great force is applied to one end of the slitted pipe 51, it can be further prevented from being immediately transmitted to the other end and in that torque transmissibility to the other end of the slitted pipe 51 can be enhanced by further rotating the slitted pipe 51.

Some modifications of the slitted pipe 51 of the eighth embodiment are conceivable. For example, the structure of the above-described sixth embodiment can be added to the structure of the slitted pipe 51. That is, the pitch of the helical slit pattern 55 extending in the longitudinal direction of the tubular member 53 may gradually decrease from one end toward the other end of the slitted pipe 51.

Alternatively, the structure of the above-described seventh embodiment can be added to the structure of the slitted pipe 51. That is, the pitch of the helical slit pattern 55 extending in the longitudinal direction of the tubular member 53 may decrease in a stepwise manner from one end toward the other end of the slitted pipe 51.

### Ninth Embodiment

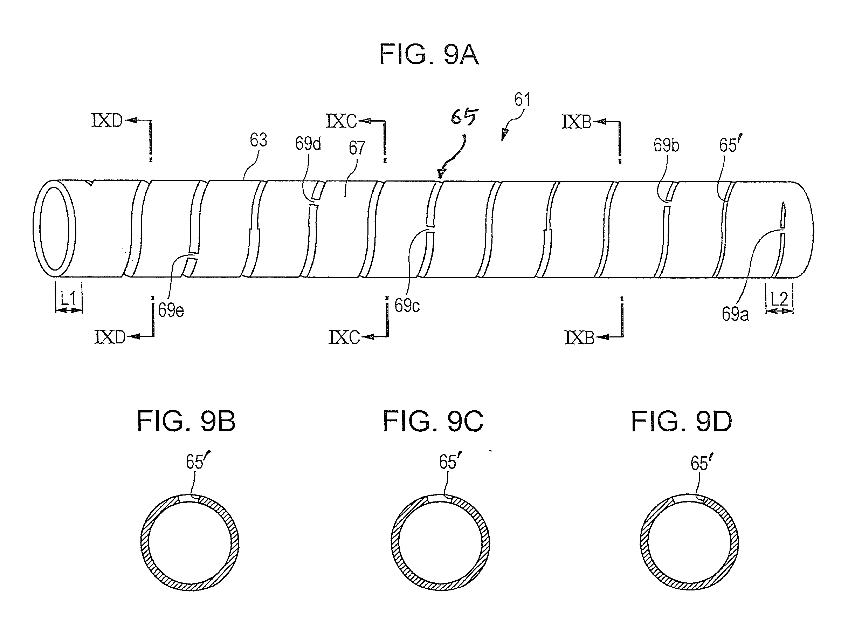

Fig. 9A is an overall perspective view of a slitted pipe according to a ninth embodiment of the present invention, Fig. 9B is a cross-sectional view taken along line IXB-IXB of Fig. 9A, Fig. 9C is a cross-sectional view taken along line IXC-IXC of Fig. 9A, and Fig. 9D is a cross-sectional view taken along line IXD-IXD of Fig. 9A.

Referring to Figs. 9A to 9D, a slitted pipe 61 of the ninth embodiment is different from the above-described first to eighth embodiments in that the slit width of one helical slit pattern extending in a longitudinal direction of a tubular member 63 increases in a stepwise manner from one end toward the other end. That is, the slitted pipe 61 includes a helical slit pattern 65 extending in the longitudinal direction of the tubular member 53, a belt-like helical portion 67 defined by the slit pattern 65, and girders 69a, 69b, 69c, 69d, 69e, ... provided on the helical slit pattern 65 to divide the slit pattern 65 into a plurality of slits 65'. The width of the slit 65' increases in three steps from one end (a right end in Fig. 9A) toward the other end (a left end in Fig. 9A) of the slitted pipe 61. According to this slitted pipe 61, flexibility at the other end (left end) of the slitted pipe 61 can be enhanced. Hence, when the slitted pipe 61 is inserted in a blood vessel of a patient, perforation of the blood vessel can be further prevented. Moreover, the base point can be easily formed when the slitted pipe 61 is curved.

In the ninth embodiment, the slit pattern 65 is also not formed within a distance L1 from the left end of the slitted pipe 61 and a distance L2 from the right end of the slitted pipe 61. Hence, the slitted pipe 61 is not divided by the slit pattern 65, but can be handled as one piece. This allows the slitted pipe 61 to be handled easily.

The slitted pipe 61 is similar to the slitted pipe 1, the slitted pipe 11, the slitted pipe 21, the slitted pipe 31, the slitted pipe 41, the slitted pipe 51, the slitted pipe 71, and the slitted pipe 81 in that, even if great force is suddenly applied to one end of the slitted pipe 61, it can be further prevented from being immediately transmitted to the other end and in that torque transmissibility to the other end of the slitted pipe 61 can be enhanced by further rotating the slitted pipe 61.

Some modifications of the slitted pipe 61 of the ninth embodiment are also conceivable. For example, the structure of the above-described sixth embodiment can be added to the structure of the slitted pipe 61. That is, the pitch of the helical slit pattern 65 extending in the longitudinal direction of the tubular member 63 may gradually decrease from one end toward the other end of the slitted pipe 61.

Alternatively, the structure of the above-described seventh embodiment can be added to the structure of the slitted pipe 61. That is, the pitch of the helical slit pattern 65 extending in the longitudinal direction of the tubular member 63 may decrease in a stepwise manner from one end toward the other end of the slitted pipe 61. Tenth Embodiment

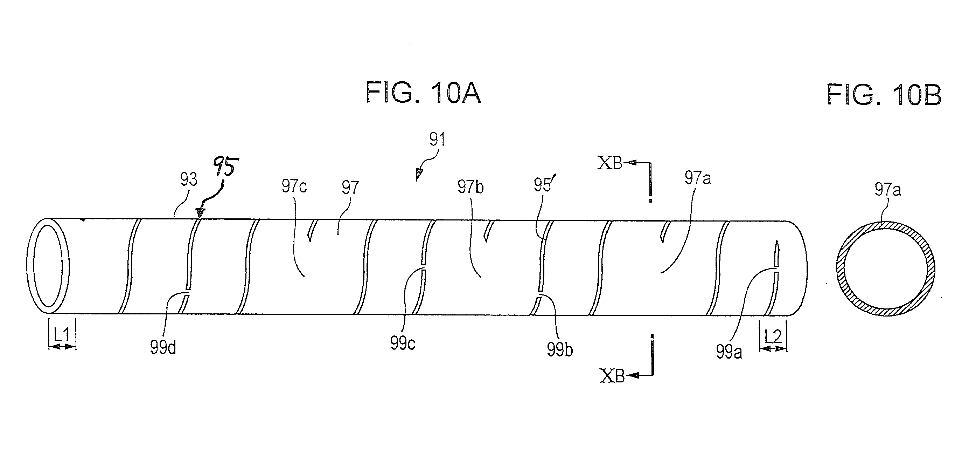

Fig. 10A is an overall perspective view of a slitted pipe according to a tenth embodiment of the present invention, and Fig. 10B is a cross-sectional view taken along line XB-XB of Fig. 10A.

Referring to Figs. 10A and 10B, a slitted pipe 91 of the tenth embodiment is different from the above-described first to ninth embodiments in that cylindrical girders are formed at three positions other than both longitudinal end portions of the slitted pipe 91 and a slits are not formed within ranges of the girders. That is, the slitted pipe 91 includes a helical slit pattern 95 extending in a longitudinal direction of a tubular member 93, a belt-like helical portion 97 defined by the slit pattern 95, and girders 99a, 99b, 99c, 99d, ... provided on the helical slit pattern 95 to divide the slit pattern 95 into a plurality of slits 95'. Cylindrical girders 97a, 97b, and 97c are provided at three positions other than both longitudinal end portions of the slitted pipe 91. According to this slitted pipe 91, torque transmissibility can be enhanced further.

In the tenth embodiment, the slit pattern 95 is also not formed within a distance L1 from a left end of the slitted pipe 91 and a distance L2 from a right end of the slitted pipe 91. Hence, the slitted pipe 91 is not divided by the slit pattern 95, but can be handled as one piece. This allows the slitted pipe 91 to be handled easily.

The slitted pipe 91 is similar to the slitted pipe 1, the slitted pipe 11, the slitted pipe 21, the slitted pipe 31, the slitted pipe 41, the slitted pipe 51, the slitted pipe 61, the slitted pipe 71, and the slitted pipe 81 in that, even if great force is suddenly applied to one end of the slitted pipe 91, it can be further prevented from immediately transmitted to the other end and in that torque transmissibility to the other end of the slitted pipe 91 can be enhanced by further rotating the slitted pipe 91. Eleventh Embodiment

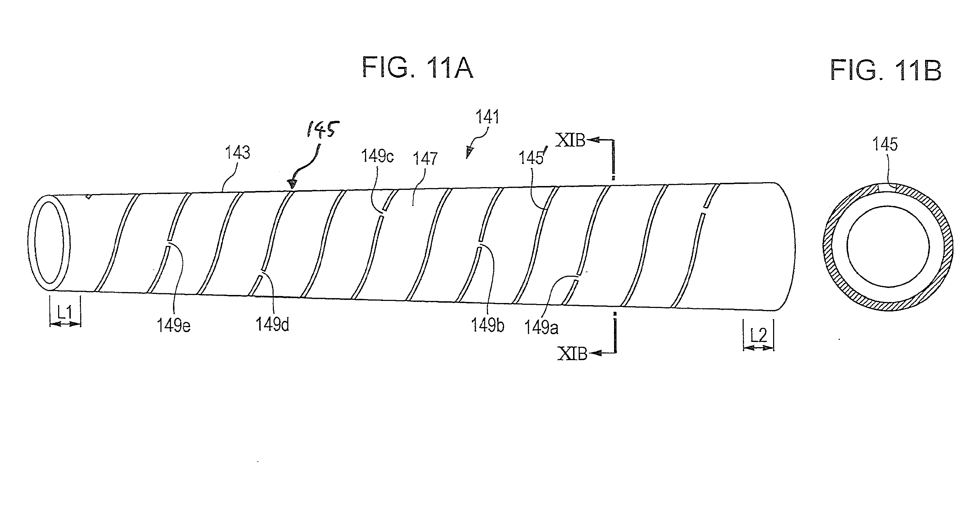

Fig. 11A is an overall perspective view of a slitted pipe according to an eleventh embodiment of the present invention, and Fig. 11B is a cross-sectional view taken along line XIB-XIB of Fig. 11A.

Referring to Figs. 11A and 11B, a slitted pipe 141 of the eleventh embodiment is different from the above-described first to tenth embodiments in that the slitted pipe 141 is tapered such that the outer diameter thereof gradually decreases from one end (a right end in Fig. 11A) toward the other end (a left end in Fig. 11A). That is, the slitted pipe 141 includes a helical slit pattern 145 extending in a longitudinal direction of a tubular member 143, a belt-like helical portion 147 defined by the slit pattern 145, and girders 149a, 149b, 149c, 149d, 149e, ... provided on the helical slit pattern 145 to divide the slit pattern 145 into a plurality of slits 145'. The outer diameter of the slitted pipe 141 gradually decreases from one end (right end) toward the other end (left end). According to this slitted pipe 141, flexibility at the other end (left end) of the slitted pipe 141 can be enhanced further.

Modifications of the slitted pipe 141 of the eleventh embodiment are also conceivable. For example, the outer diameter of the slitted pipe 141 can decrease in a stepwise manner from one end (right end) toward the other end (left end). In this case, since the outer diameter decreases in a stepwise manner from one end toward the other end, flexibility at the other end (left end) of the slitted pipe 141 can be further enhanced, and the slitted pipe 141 can be easily produced, for example, by electropolishing.

In the eleventh embodiment, the slit pattern 145 is also not formed within a distance L1 from the left end of the slitted pipe 141 and a distance L2 from the right end of the slitted pipe 141. Hence, the slitted pipe 141 is not divided by the slit pattern 145, but can be handled as one piece. This allows the slitted pipe 141 to be handled easily.

The slitted pipe 141 is similar to the slitted pipe 1, the slitted pipe 11, the slitted pipe 21, the slitted pipe 31, the slitted pipe 41, the slitted pipe 51, the slitted pipe 61, the slitted pipe 71, the slitted pipe 81, and the slitted pipe 91 in that, even if great force is suddenly applied to one end of the slitted pipe 141, it can be further prevented from immediately transmitted to the other end and in that torque transmissibility to the other end of the slitted pipe 141 can be enhanced by further rotating the slitted pipe 141.

Cases in which the slitted pipes of the above-described first to eleventh embodiments are applied to guidewires will be described below with reference to the drawings.

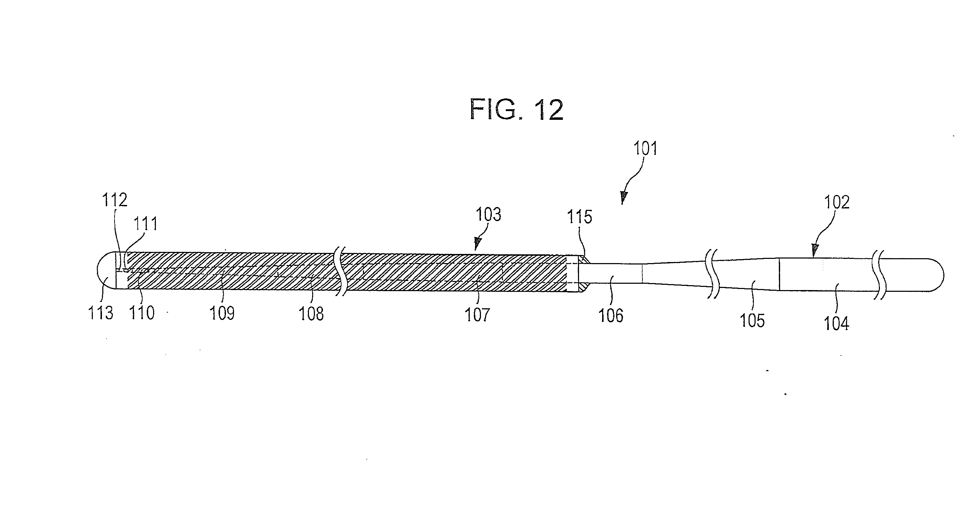

In Figs. 12 to 14, for easy understanding, the slitted pipe is schematically illustrated as a whole while being shortened in a longitudinal direction. Hence, overall dimensions of the slitted pipe are different from actual ones.

### Twelfth Embodiment

Fig. 12 is an overall view of a guidewire according to a twelfth embodiment of the present invention. A guidewire 101 of the twelfth embodiment illustrated in Fig. 12 is used, for example, for medical treatment of a blood vessel of the heart. In the twelfth embodiment, the guidewire 101 is about 1900 mm in length. The guidewire 101 includes a core shaft 102, a slitted pipe 103 according to any of the first to eleventh embodiments, a distal-end brazing part 113 that joins a distal end of the core shaft 102 and a distal end of the slitted pipe 103, and a proximal-end brazing part 115 that joins the core shaft 102 and a proximal end of the slitted pipe 103.

The core shaft 102 includes a first distal end portion 112 provided at the most distal end, a second distal end portion 111 adjacent to a proximal end of the first distal end portion 112, a third distal end portion 110 adjacent to a proximal end of the second distal end portion 111, a first tapered portion 109 adjacent to a proximal end of the third distal end portion 110, a second tapered portion 108 adjacent to a proximal end of the first tapered portion 109, a third tapered portion 107 adjacent to a proximal end of the second tapered portion 108, a first cylindrical portion 106 adjacent to a proximal end of the third tapered portion 107, a fourth tapered portion 105 adjacent to a proximal end of the first cylindrical portion 106, and a cylindrical proximal end portion 104 adjacent to a proximal end of the fourth tapered portion 105.

An outer surface of a section extending from the distal end of the guidewire 101 to the fourth tapered portion 105 of the core shaft 102 via the slitted pipe 103 is coated with a hydrophilic coating agent.

While the material of the core shaft 102 is not particularly limited, stainless steel (SUS304) is used in the twelfth embodiment. As other materials, a superelastic alloy, such as a Ni-Ti alloy, and a piano wire are used.

Examples of hydrophilic materials are a cellulosic high polymer, a polyethylene oxide high polymer, a maleic anhydride high polymer (for example, a maleic anhydride copolymer such as a methyl vinyl ether-maleic anhydride copolymer), an acrylamide high polymer (for example, a block copolymer of polyacrylamide and poly(glycidyl methacrylate-dimethylacrylamide), hydrosoluble nylon, polyvinyl alcohol, polyvinylpyrrolidone, and hyaluronate. In the twelfth embodiment, hyaluronate is used as the hydrophilic material.

Since the guidewire 101 of the twelfth embodiment includes the core shaft 102 and the slitted pipe 103 covering a distal end portion of the core shaft 102, it is easy to fix the slitted pipe 103, which has no slits in both end portions, to the core shaft 102, and therefore, the guidewire 101 itself can be produced easily.

Further, according to the guidewire 101 of the twelfth embodiment, torque transmissibility of the guidewire 101 in rotation in at least one direction can be enhanced.

In the slitted pipe according to any of the above-described fourth, sixth to ninth, and eleventh embodiments, the other end of the slitted pipe is preferably located on a distal side of the core shaft. In this case, the guidewire can be easily produced, torque transmissibility of the guidewire in rotation in at least one direction can be enhanced, and flexibility at the distal end of the guidewire can be further enhanced by the slitted pipe.

Preferably, a gap is formed between the core shaft 102 and the slitted pipe 103. In this case, flexibility can be further enhanced when the guidewire is curved, and this allows the guidewire to be easily inserted to an end portion of the blood vessel.

### Thirteenth Embodiment

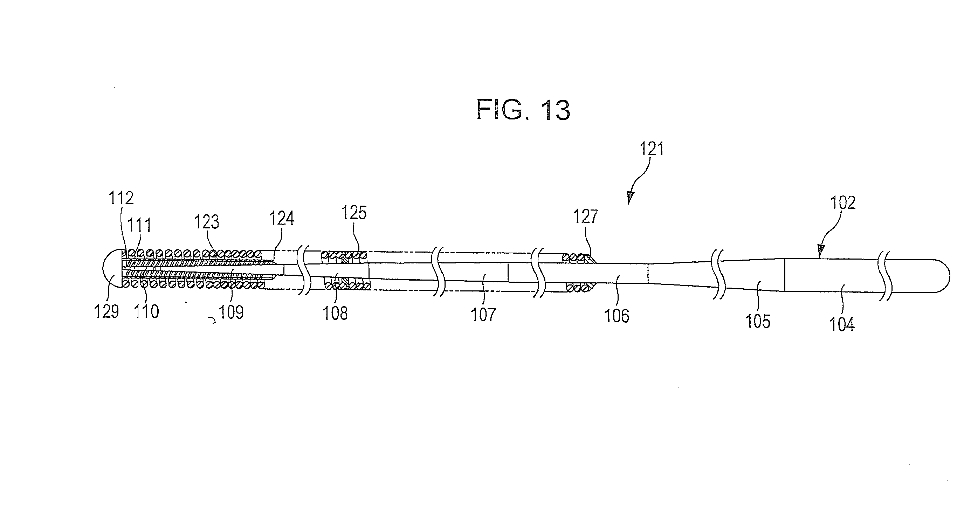

Fig. 13 is an overall view of a guidewire according to a thirteenth embodiment of the present invention.

In Fig. 13, the same structures as those illustrated in Fig. 12 are denoted by the same reference numerals, and descriptions thereof are skipped.

Referring to Fig. 13, a guidewire 121 according to the thirteenth embodiment includes a core shaft 102, an outer coil 125 covering a distal end portion of the core shaft 102, a slitted pipe 123 according to any of the first to tenth embodiments, which is disposed in the outer coil 125 to cover the distal end portion of the core shaft 102, a distal-end brazing part 129 that joins a distal end of the core shaft 102, a distal end of the outer coil 125, and a distal end of the slitted pipe 123, an inner proximal-end brazing part 124 that joins a proximal end of the slitted pipe 123 and the core shaft 102, and a proximal-end brazing part 127 that joins the core shaft 102 and a proximal end of the outer coil 125.

An outer surface of a section extending from the distal end of the guidewire 121 to a fourth tapered portion 105 of the core shaft 102 via the outer coil 125 is coated with a hydrophilic coating agent.

The guidewire 121 of the thirteenth embodiment includes the core shaft 102, the outer coil 125 covering the distal end portion of the core shaft 102, and the slitted pipe 123 disposed in the outer coil 125 to cover the distal end portion of the core shaft 102. Hence, the guidewire 121 can be easily produced, and torque transmissibility of the guidewire 121 in rotation in at least one direction can be enhanced. Moreover, flexibility of the guidewire 121 can be adjusted by the slitted pipe 123 disposed in the outer coil 125 while the outer diameter of the outer coil 125 is maintained.

In the slitted pipe according to any of the fourth, sixth to ninth, and eleventh embodiments, the other end of the slitted pipe is preferably located on a distal side of the core shaft. In this case, the guidewire 121 can be easily produced, and torque transmissibility of the guidewire 121 in rotation in at least one direction can be enhanced. Moreover, flexibility at the distal end of the guidewire 121 can be further enhanced by the slitted pipe 123 disposed in the outer coil 125 while the outer diameter of the outer coil 125 is maintained.

Preferably, gaps are formed between the core shaft 102 and the slitted pipe 123 and between the outer coil 125 and the slitted pipe 123. In this case, flexibility can be enhanced when the guidewire 121 is curved, and this allows the guidewire 121 to be easily inserted to an end portion of the blood vessel.

### Fourteenth Embodiment

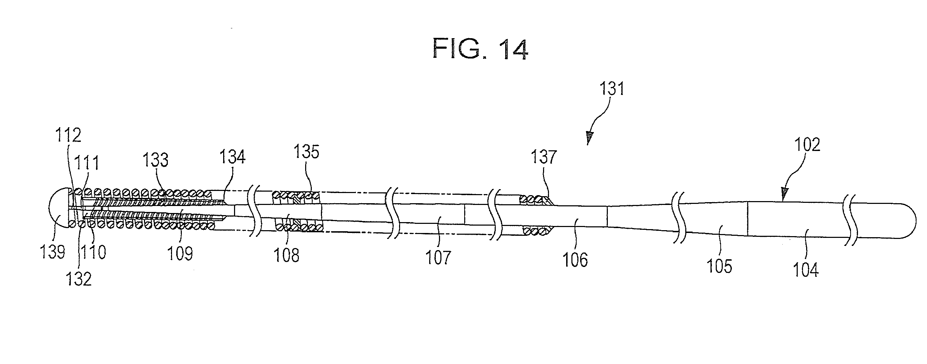

Fig. 14 is an overall view of a guidewire according to a fourteenth embodiment of the present invention.

In Fig. 14, the same structures as those illustrated in Fig. 12 are denoted by the same reference numerals, and descriptions thereof are skipped.

Referring to Fig. 14, a guidewire 131 of the fourteenth embodiment includes a core shaft 102, an outer coil 135 covering a distal end portion of the core shaft 102, a slitted pipe 133 according to any of the first to tenth embodiments, which is disposed in the outer coil 135 to cover the distal end portion of the core shaft 102, a distal-end brazing part 139 that joins a distal end of the core shaft 102 and a distal end of the outer coil 135, an inner distal-end brazing part 132 that joins a distal end of the slitted pipe 133 and the core shaft 102, an inner proximal-end brazing part 134 that joins a proximal end of the slitted pipe 133 and the core shaft 102, and a proximal-end brazing part 137 that joins the core shaft 102 and a proximal end of the outer coil 135.

In the fourteenth embodiment, the distal end of the slitted pipe 133 is located a predetermined distance apart from the distal end of the core shaft 102 toward the proximal end. Further, an outer surface of a section extending from the distal end of the guidewire 131 to a fourth tapered portion 105 of the core shaft 102 via the outer coil 135 is coated with a hydrophilic coating agent.

According to the guidewire 131 of the fourteenth embodiment, since the distal end of the slitted pipe 133 is located a predetermined distance apart from the distal end of the core shaft 102 toward the proximal end, the distal end portion of the guidewire 131 can be easily bent, and vascular selectivity of the guidewire 131 can be enhanced.

Preferably, gaps are formed between the core shaft 102 and the slitted pipe 133 and between the outer coil 135 and the slitted pipe 133. In this case, flexibility can be enhanced when the guidewire 131 is curved, and this allows the guidewire 131 to be easily inserted to an end portion of the blood vessel.

## Claims

1. A slitted pipe (1; 11; 71; 21; 81; 31; 41; 51; 61; 91; 141) comprising:
a slit pattern (5; 15; 75; 25; 85; 25; 85; 35; 45; 55; 65; 95; 145) provided in an area except for both end portions in a longitudinal direction of the slitted pipe to extend in the longitudinal direction in a helical shape; and
a plurality of girders (9a to 9f; 19a to 19f; 79a to 79f; 29a to 29f; 89a to 89e; 39a to 39f; 49a to 49f; 59a to 59f; 69a to 69e; 99a to 99d; 149a to 149e) provided on the slit pattern (5; 15; 75; 25; 85; 25; 85; 35; 45; 55; 65; 95; 145)to divide the slit pattern (5; 15; 75; 25; 85; 25; 85; 35; 45; 55; 65; 95; 145) into a plurality of slits (5'; 15'; 75'; 25'; 85'; 25'; 85'; 35'; 45'; 55'; 65'; 95'; 145').

2. The slitted pipe according to Claim 1, wherein girders (9a to 9f) adjacent in the longitudinal direction are arranged while being shifted from each other by a predetermined angle in cross section per predetermined distance in the longitudinal direction.

3. The slitted pipe according to Claim 1 or 2, wherein the girders (89a to 89e) provided on the slit pattern (85) are arranged to form at least one helical shape different from the helical shape of the slit pattern (85).

4. The slitted pipe according to any one of Claims 1 to 3, wherein a length of the girders (79a to 79f) along the helical shape differs according to rigidity in the longitudinal direction.

5. The slitted pipe according to any one of Claims 1 to 3, wherein a ratio of a length of the girders along the helical shape to a length of the slit (75') along the helical shape per unit length differs according to rigidity in the longitudinal direction.

6. The slitted pipe according to Claim 4 or 5, wherein a ratio of the length of the girders along the helical shape to a length of the slit (25') along the helical shape decreases from one end toward the other end in the longitudinal direction.

7. The slitted pipe according to any one of Claims 1 to 6, wherein a pitch of the slit pattern (35) gradually decreases from one end toward the other end in the longitudinal direction.

8. The slitted pipe according to any one of Claims 1 to 6, wherein a pitch of the slit pattern (45) decreases in a stepwise manner from one end toward the other end in the longitudinal direction.

9. The slitted pipe according to any one of Claims 1 to 8, wherein a width of the slit (55') gradually increases from one end toward the other end in the longitudinal direction.

10. The slitted pipe according to any one of Claims 1 to 8, wherein a width of the slit (65') increases in a stepwise manner from one end toward the other end in the longitudinal direction.

11. The slitted pipe according to any one of Claims 1 to 10, wherein the girders (97a, 97b, 97c) are cylindrically formed at at least one position other than the end portions in the longitudinal directions.

12. The slitted pipe according to any one of Claims 1 to 11, wherein the slitted pipe is circular in cross section.

13. The slitted pipe according to any one of Claims 1 to 12, wherein the slitted pipe is tapered such that an outer diameter of the slitted pipe gradually decreases from one end toward the other end in the longitudinal direction.

14. The slitted pipe according to any one of Claims 1 to 12, wherein an outer diameter of the slitted pipe decreases in a stepwise manner from one end toward the other end in the longitudinal direction.

15. A guidewire (101; 121; 131) comprising:
a core shaft (102); and
a slitted pipe (103; 123; 133) according to any one of Claims 1 to 14, the slitted pipe (103; 123; 133) covering a distal end portion of the core shaft (102).

16. The guidewire (101; 121; 131) according to Claim 15, wherein a gap is formed between the core shaft (102) and the slitted pipe (103; 123; 133).

17. The guidewire (101; 121; 131) according to claim 15 or 16, comprising:
a slitted pipe according to any one of Claims 6 to 10, the slitted pipe(103; 123; 133) covering the distal end portion of the core shaft (102) such that the other end of the slitted pipe(103; 123; 133) is located on a distal side of the core shaft (102).

18. The guidewire (121; 131) according to any one of Claims 15 to 17, further comprising:
a coil body (125) covering a distal end portion of the core shaft (102);
wherein the slitted pipe(123; 133) is disposed in the coil body (125) to cover the distal end portion of the core shaft (102).

19. The guidewire (121; 131) according to Claim 18, wherein gaps are formed between the core shaft (102) and the slitted pipe (123; 133) and between the coil body (125) and the slitted pipe (123; 133).

20. The guidewire (121; 131) according to Claim 18 or 19, comprising
a slitted pipe according to any one of Claims 6 to 10, wherein the slitted pipe (123; 133) is disposed in the coil body (125) to cover the distal end portion of the core shaft (102) such that the other end of the slitted pipe (121; 131) is located on a distal side of the core shaft (102).

21. The guidewire (131) according to any one of Claims 18 to 20, wherein a distal end of the slitted pipe (133) is located a predetermined distance apart from a distal end of the core shaft (102) toward a proximal end.
